# EUROPEAN PATENT APPLICATION

(11) **EP 2 336 311 A1**
(43) Date of publication of application: **22.06.2011**
(21) Application number: 09815887.6
(22) Date of filing: 25.09.2009
(51) Int. Cl.: C12N 15/00, C07K 16/18, C12N 5/10, C12N 15/02, C12N 15/09, G01N 33/53

(54) **ANTI-TUNA VASA ANTIBODY**

(30) Priority: 25.09.2008 JP 2008246963
(71) Applicant: National University Corporation Tokyo University of Marine Science And Technology, Minato-ku Tokyo 108-8477 (JP); Nippon Suisan Kaisha, Ltd., Chiyoda-ku Tokyo 100-8686 (JP)
(72) Inventor: YOSHIZAKI, Goro, Tokyo 108-8477 (JP); TAKEUCHI, Yutaka, Tokyo 108-8477 (JP); MIWA, Misako, Tokyo 108-8477 (JP); KABEYA, Naoki, Tokyo 108-8477 (JP)
(74) Representative: Richards, William John
(86) International application number: PCT/JP2009/004837
(87) International publication number: WO 2010/035465

(57) **Abstract**

An object of the present invention is to provide a means for distinguishing between a germ cell derived from a donor (tuna) and a germ cell derived from a recipient in a method for inducing differentiation of a tuna germ cell, wherein a primordial germ cell derived from the tuna is transplanted into an early embryo of the heterologous recipient fish. The present inventors compared a Vasa amino acid sequence of bluefin tuna with those of other fish (black skipjack, skipjack, chub mackerel, blue mackerel, round frigate mackerel and frigate mackerel), identified amino acid sequence regions specific to bluefin tuna, and, by using the amino acid sequences specific to bluefin tuna as antigens, successfully produced monoclonal antibodies specifically recognizing primordial germ cells, spermatogonia, oogonia or oocytes derived from bluefin tuna, thus accomplishing the present invention.

SEQUENCE LISTING

<110> National University Corporation Tokyo University of Marine Science and Technology
Nagasaki Prefecture
Nippon Suisan Kaisha, Ltd.

<120> anti-Bluefin tuna Vasa antibody

<130> F2008-005

<150> JP2008-246963
<151> 2008-09-25

<160> 13

<170> PatentIn version 3.1

<210> 1
<211> 12
<212> PRT
<213> Thunnus thynnus

<400> 1

Thr Ser Thr Ile Thr Leu Thr Ser Arg Thr Ser Ser
1               5                   10

<210> 2
<211> 10
<212> PRT
<213> Thunnus thynnus

<400> 2

Phe Trp Asn Thr Asn Gly Gly Glu Phe Gly
1               5                   10

<210> 3
<211> 10
<212> PRT
<213> Thunnus thynnus

<400> 3

Cys Arg Met Asp Gln Ser Glu Phe Asn Gly
1               5                   10

<210> 4
<211> 11
<212> PRT
<213> Thunnus thynnus

<400> 4

Asp Asn Gly Met Arg Glu Asn Gly Tyr Arg Gly
1               5                   10

<210> 5
<211> 14
<212> PRT
<213> Thunnus thynnus

<400> 5

Gly Phe Ser Gln Gly Gly Asp Gln Gly Gly Arg Gly Gly Phe
1               5                   10

<210> 6
<211> 15
<212> PRT
<213> Thunnus thynnus

<400> 6

Thr Arg Gly Glu Asp Lys Asp Pro Glu Lys Lys Asp Asp Ser Asp
1               5                   10                  15

<210> 7
<211> 10
<212> PRT
<213> Thunnus thynnus

<400> 7

Ala Asp Gly Gln Leu Ala Arg Ser Leu Val
1               5                   10

<210> 8
<211> 12
<212> PRT
<213> Thunnus thynnus

<400> 8

Pro Ala Thr Thr Gly Phe Asn Pro Pro Arg Lys Asn
1               5                   10

<210> 9
<211> 22
<212> PRT
<213> Thunnus thynnus

<400> 9

Arg Gly Ser Phe Gln Asp Asn Ser Val Lys Ser Gln Pro Ala Val Gln
1               5                   10                  15

Thr Ala Ala Asp Asp Asp
            20

<210>  10
<211>  644
<212>  PRT
<213>  Thunnus thynnus

<400>  10

Met Asp Glu Trp Glu Glu Glu Gly Asn Thr Ser Thr Ile Thr Leu Thr
1               5                   10                  15

Ser Arg Thr Ser Ser Glu Gly Thr Gln Gly Asp Phe Trp Asn Thr Asn
            20                  25                  30

Gly Gly Glu Phe Gly Arg Gly Arg Gly Gly Arg Gly Arg Gly Arg Gly
        35                  40                  45

Gly Phe Lys Ser Ser Tyr Ser Ser Gly Gly Asp Gly Asn Asp Glu Asp
    50                  55                  60

Lys Trp Asn Asn Ala Gly Gly Glu Arg Gly Gly Phe Arg Gly Arg Gly
65                  70                  75                  80

Gly Gln Gly Arg Gly Arg Gly Phe Cys Arg Met Asp Gln Ser Glu Phe
                85                  90                  95

Asn Gly Asp Asp Asn Gly Met Arg Glu Asn Gly Tyr Arg Gly Gly Arg
            100                 105                 110

Gly Gly Arg Gly Arg Gly Gly Gly Phe Ser Gln Gly Gly Asp Gln Gly
        115                 120                 125

Gly Arg Gly Gly Phe Gly Gly Gly Tyr Arg Gly Lys Asp Glu Glu Ile
    130                 135                 140

Phe Thr Arg Gly Glu Asp Lys Asp Pro Glu Lys Lys Asp Asp Ser Asp
145                 150                 155                 160

Arg Pro Lys Ile Thr Tyr Val Pro Pro Thr Leu Pro Glu Asp Glu Asp

                165                 170                 175

Ser Ile Phe Ser His Tyr Glu Thr Gly Ile Asn Phe Asp Lys Tyr Asp
            180                 185                 190

Asp Ile Met Val Asp Val Ser Gly Thr Asn Pro Pro Gln Ala Val Met
        195                 200                 205

Thr Phe Asp Glu Ala Ala Leu Cys Glu Ser Leu Arg Lys Asn Val Ser
    210                 215                 220

Lys Ser Gly Tyr Val Lys Pro Thr Pro Val Gln Lys His Gly Ile Pro
225                 230                 235                 240

Ile Ile Ser Ala Gly Arg Asp Leu Met Ala Cys Ala Gln Thr Gly Ser
                245                 250                 255

Gly Lys Thr Ala Ala Phe Leu Leu Pro Ile Leu Gln Gln Leu Met Ala
            260                 265                 270

Asp Gly Val Ala Ala Ser Arg Phe Ser Glu Leu Gln Glu Pro Glu Ala
        275                 280                 285

Ile Ile Val Ala Pro Thr Arg Glu Leu Ile Asn Gln Ile Tyr Leu Glu
    290                 295                 300

Ala Arg Lys Phe Ala Phe Gly Thr Cys Val Arg Pro Val Val Val Tyr
305                 310                 315                 320

Gly Gly Val Ser Thr Gly His Gln Ile Arg Glu Ile Glu Arg Gly Cys
                325                 330                 335

Asn Val Val Cys Gly Thr Pro Gly Arg Leu Leu Asp Met Ile Gly Arg
            340                 345                 350

Gly Lys Val Gly Leu Ser Lys Leu Arg Tyr Leu Val Leu Asp Glu Ala
        355                 360                 365

Asp Arg Met Leu Asp Met Gly Phe Glu Pro Asp Met Arg Arg Leu Val
    370                 375                 380

Gly Ser Pro Gly Met Pro Ser Lys Glu Asn Arg Gln Thr Leu Met Phe

385                 390                 395                 400

Ser Ala Thr Tyr Pro Glu Asp Ile Gln Arg Met Ala Ala Asp Phe Leu
                405                 410                 415

Lys Thr Asp Tyr Leu Phe Leu Ala Val Gly Val Val Gly Gly Ala Cys
            420                 425                 430

Ser Asp Val Glu Gln Thr Phe Ile Gln Val Thr Lys Phe Ser Lys Arg
        435                 440                 445

Glu Gln Leu Leu Asp Leu Leu Lys Thr Thr Gly Thr Glu Arg Thr Met
    450                 455                 460

Val Phe Val Glu Thr Lys Arg Gln Ala Asp Phe Ile Ala Thr Phe Leu
465                 470                 475                 480

Cys Gln Glu Lys Val Pro Thr Thr Ser Ile His Gly Asp Arg Glu Gln
                485                 490                 495

Arg Glu Arg Glu Gln Ala Leu Ala Asp Phe Arg Ser Gly Lys Cys Pro
            500                 505                 510

Val Leu Val Ala Thr Ser Val Ala Ala Arg Gly Leu Asp Ile Pro Asp
        515                 520                 525

Val Gln His Val Val Asn Phe Asp Leu Pro Asn Asn Ile Asp Glu Tyr
    530                 535                 540

Val His Arg Ile Gly Arg Thr Gly Arg Cys Gly Asn Thr Gly Arg Ala
545                 550                 555                 560

Val Ser Phe Tyr Asp Pro Asp Ala Asp Gly Gln Leu Ala Arg Ser Leu
                565                 570                 575

Val Thr Val Leu Ser Lys Ala Gln Gln Glu Val Pro Ser Trp Leu Glu
            580                 585                 590

Glu Ser Ala Phe Ser Gly Pro Ala Thr Thr Gly Phe Asn Pro Pro Arg
        595                 600                 605

Lys Asn Phe Ala Ser Thr Asp Ser Arg Lys Arg Gly Ser Phe Gln Asp

610                615                620

Asn Ser Val Lys Ser Gln Pro Ala Val Gln Thr Ala Ala Asp Asp Asp
625                630                635                640

Glu Glu Trp Glu

<210> 11
<211> 2394
<212> DNA
<213> Thunnus thynnus

<400> 11
ctacacaaat cagcgaccgg actgataaca ccatagactt ctgcttgaga agcttttgat     60

aagtgaaaaa agatggatga gtgggaagaa gagggaaata ctagcactat tacactaacc    120

agccgcacct caagtgaagg cacacaagga gacttctgga acaccaatgg tggtgaattt    180

ggaaggggtc gcggtggaag aggcagaggg agaggaggat ttaaaagctc atactcctca    240

ggtggagatg ggaatgatga ggacaaatgg aacaatgcag gaggagaaag aggtggtttc    300

agaggtagag gaggccaagg gcgcggcaga ggattttgca gaatggatca aagtgaattc    360

aatggagatg acaatggaat gcgtgaaaat gggtatagag gaggaagagg gggcagagga    420

agaggaggag gtttcagcca aggtggcgac cagggtggca gaggaggctt tggaggaggt    480

tatcgtggaa aagatgagga gatctttact cgaggggaag ataaagatcc agaaaagaag    540

gacgatagtg acagaccaaa gatcacatat gttcccccaa ccctccctga agatgaggac    600

tccatttttt cccactatga aacagggatc aactttgaca agtatgatga catcatggtg    660

gatgttagtg gaaccaatcc accacaagct gtcatgactt ttgatgaggc agcattgtgc    720

gagtccctga gaaaaaacgt cagcaagtct ggttatgtga agccgacccc tgtgcagaag    780

cacggcatcc caatcatctc tgctggtaga gatctcatgg cctgtgccca gactggatct    840

ggtaaaacgg ctgcattcct gctccctatt ctgcagcagc tgatggcaga tggtgtggca    900

gcaagtcgct tcagcgagct gcaggagcct gaagcaatca ttgtggcccc aaccagggag    960

ctcatcaacc agatttacct ggaggccagg aagtttgcct ttgggacatg tgtgcgtcca   1020

gtggtggttt atggtggagt cagcactgga caccaaataa gagaaatcga aaggggatgc   1080

aatgtagtgt gtggaacacc agggaggcta ttggatatga ttggaagagg aaaggttggg   1140

ttgagtaagc tgcggtactt ggtgctagat gaggccgacc ggatgttgga tatgggattt   1200

gagcctgaca tgcgccgcct ggtgggctca cctggaatgc catccaaaga gaaccgtcag 1260
actctgatgt tcagtgccac ataccctgaa gacatccaga ggatggcggc tgacttcctc 1320
aagacagact atttgttcct ggctgtgggt gtggtgggtg gagcctgcag tgatgtggag 1380
cagacattta tccaagtcac aaagttctcc aagagagagc agctccttga cctcctgaag 1440
accactggaa cggagcgcac catggtgttt gtagagacca aacgacaagc tgattttatt 1500
gccacgttct tgtgccaaga gaaagttcca actaccagca ttcacggtga ccgagagcag 1560
cgggagcgag agcaggctct ggcagacttc cgctctggta aatgtccagt cctagtagca 1620
acctctgtag ctgcccgcgg tctggatatt ccagatgtac agcatgtggt taactttgac 1680
ctccccaaca acattgatga atatgtccac cgtattggga ggactggccg ctgcggtaac 1740
acagggaggg cagtgtcttt ctatgaccct gatgctgatg gccaactggc tcgctccttg 1800
gtcacagtcc tgtccaaggc ccagcaggaa gtgccttcat ggttagaaga gtctgcgttc 1860
agcggacctg ctaccactgg ctttaaccca cctaggaaga actttgcctc cacagactcc 1920
aggaagagag gatctttcca agacaacagt gtgaagagcc agccggctgt tcagactgca 1980
gcggatgatg atgaggaatg ggaatagagg agcagcacac ccacacagca ttgacctgag 2040
ttgcttttta ttttcaggtg ttcagtttgt tgtagtttta tcacgtttct gtttgaatat 2100
agaaaaagtt tgtctcatgc cggacaaagt taaaaatgtc aagtgaggtg ttaaatggga 2160
aaaccagttt ttttttgtgt gatctgtcat ttccattctt cactgactgg cattttgtga 2220
agtttgtttt attttttatt gttttaatca tcacttgcat ttaaaatgtt taaaaaagga 2280
aactgtgtct gacgaccaaa aagtaaaact tataaatgtc aattatattt tgttttctac 2340
tcagaaaaag atcaataaat atttgttcaa agcaaaaaaa aaaaaaaaaa aaaa 2394

<210> 12
<211> 34
<212> DNA
<213> Artificial

<220>
<223> Fw primer

<400> 12
gccgggatcc agcactatta cactaaccag ccgc 34


<210> 13
<211> 34

<212> DNA
<213> Artificial

<220>
<223> Rv primer

<400> 13
gccgaagctt gctgaaacct cctcctcttc ctct 34

## Description

### Technical Field

The present invention relates to an anti-tuna Vasa antibody capable of specifically detecting a germ cell derived from a tuna, and a method for detecting a germ cell of a tuna using the anti-tuna Vasa antibody. By using this method for detecting a germ cell of a tuna, a germ cell derived from a tuna transplanted into a heterologous recipient fish can be evaluated for its incorporation into the genital gland, proliferation and/or maturation.

### Background Art

The genetic analysis using Drosophila have revealed that the genes Oskar, Vasa, Tudor, and Nanos have core functions in the determination mechanism in germ cells (see, for example, Non Patent Document 1). During the ovum formation, each of these genes accumulates in pole granules, and the germ cell fate of the blastomere harboring the maternal determination factors is determined. The Vasa gene encodes an ATP-dependent RNA helicase, whose function is thought to be involved in the translational control from mRNA to protein (see, for example, Non Patent Document 2). Moreover, the structure responsible for the enzymatic function is strongly conserved in the evolution, and therefore Vasa homolog genes have been identified in a variety of multicellular animal species ranging from platyhelminth (planarian) to human.

Based on the findings mentioned above, a method for obtaining a germ cell is reported as a simple method for selecting a cell having ability to differentiate into a germ cell, using marker gene expression as indicator, without performing complicated operations such as homologous recombination. In the method, a marker gene is incorporated into a recombinant expression vector such that it is placed under the control of a promoter sequence of a mammalian-derived Vasa homolog gene, and using the marker gene expression as indicator, a cell having ability to differentiate into a germ cell is recovered from the transgenic non-human mammal transfected with the vector (see, for example, Patent Document 1).

On the other hand, as primordial germ cells are the origin cells of ova and sperms, and they develop into individuals via the processes of maturation and fertilization, also known is a method for inducing the differentiation of an isolated primordial germ cell derived from a fish into the germ cell lineage by transplanting the isolated primordial germ cell into an early embryo of a heterologous recipient fish, particularly into the inside of mesentery in the abdominal cavity of the heterologous recipient fish at an early developmental stage (see, for example, Patent Document 2).

### Citation List

### Patent Document

Patent Document 1
   Japanese Patent Laid-Open No. 2006-333762
Patent Document 2
   Japanese Patent Laid-Open No. 2003-235558

### Non Patent Document

Non Patent Document 1
   Rongo, C., et al, Development, 121, 2737-2746, 1995 Non Patent Document 2
   Liang, L., et al, Development, 120, 1201-1211, 1994

### Summary of Invention

### Problems to be Solved by the Invention

Currently, tuna is cultured mainly in the way where wild immature fish (typically, several tens to several hundred gram) are captured by fishermen and grown to large sizes. In recent years, the quantities of tuna resources have decreased and the total allowable catches of adult fish have severe settings. In such a way that depends on natural sources for the supply of immature fish, the future stable supply of seeds is not ensured. Besides, the supply of seeds in stable qualities with better culture efficiencies is expected to become possible by establishing a technique for producing artificial seeds, like those for salmon and schnapper, which will lead to the breeding by exchanging generations with selecting parents having excellent traits. However, tuna is thought to exceed several tens of kilograms when it reaches to the first maturation, although the physiology of tuna maturation has not become sufficiently clear yet. Because tuna becomes big fish, unlike other fish species, the seed production is carried out in the way where fertilized eggs spawned naturally in fish pens or partitions in a gulf are caught with a fine mesh net. For schnapper or the like, because they spawn in aquarium, an apparatus to overflow surface seawater and catch their eggs with a net can be easily made, while the operations on the sea are laborious.

Moreover, when the crossbreeding of specific individuals is desired for the purpose of bleeding or the like, the artificial extraction of spawn, in which eggs are collected by squeezing the abdomen, is performed, and sperms are also collected to perform artificial fertilization; this is not easy when parents are in large sizes like tuna. Furthermore, in the industrial point of view, profitability may increase by shifting the timing of producing seeds for the purpose of adjusting the timing of shipment, the duration of growing fish. To this end, it is required to control the timing of spawning by shifting the season of parents by adjusting water temperatures and/or light cycles in the place where the environment can be controlled; this is laborious and costly if parents are in large sizes like tuna.

The surrogate fish technique is intended to produce seeds easily at low costs by making a fish species easy for the seed production produce gametes, or spawn eggs and sperms of a fish species difficult for such seed production. For example, applying the surrogate fish technique according to above mentioned Patent Document 2 to tuna using a small fish as a recipient to mature germ cells derived from a tuna will enable seed production and complete culture in a small aquarium, and this is expected to lead to extensive labor saving and cost saving. Transplantation of isolated primordial germ cells requires the detection of proliferation of donor primordial germ cells derived from the tuna incorporated into the recipient gonad, and the ratio of germ cells derived from the recipient and the germ cells derived from the tuna. An object of the present invention is to provide a monoclonal antibody capable of specifically detecting a tuna germ cell, and a method for detecting a germ cell derived from a tuna by using thereof, in a method for inducing differentiation of a primordial germ cell into the germ cell lineage, wherein a primordial germ cell derived from a tuna is transplanted into an early embryo of the heterologous recipient fish, the antibody is capable of specifically detecting a primordial germ cell, a spermatogonium, an oogonium or an oocyte derived from the tuna, which is the donor fish, without binding to a germ cell derived from the recipient, and enables distinguishing between the germ cell derived from the tuna and the germ cell derived from the recipient.

### Means for Solving the Problems

In *Salmonidae* fish, the present inventors have succeeded in producing rainbow trout individuals from masu salmon by performing xenogeneic germ cell transplantation. In this case, it has become possible to analyze easily to determine whether a transplantation is successful or not by using a transgenic lineage in which rainbow trout germ cells are visualized with green fluorescent protein. In addition, the present inventors have already developed a method to determine whether a transplantation is successful or not without using a transgenic lineage in order to apply xenogeneic germ cell transplantation to wild endangered fish species and cultured fish species. By this method, wild type rainbow trout germ cells engrafted into the genital gland of a char host have been successfully detected. The present inventors aim for further applying xenogeneic germ cell transplantation to other marine fish. In order to accomplish this, a technique to analyze whether transplanted germ cells of a *Percomorphi* donor fish such as tuna are incorporated into the genital gland of the host and engrafted or not has been necessary.

Therefore the present inventors selected the Vasa gene from the genes known to be specifically expressed in primordial germ cells, such as Nanos, Deadend and Vasa genes, and determined the nucleotide sequences of the Vasa genes of tuna, chub mackerel, blue mackerel, black skipjack and croaker for the first time. Among these genes, the present inventors further focused on the Vasa gene of tuna, which is most likely to become a *Percomorphi* donor fish, confirmed that the tuna Vasa gene is specifically expressed in tuna primordial germ cells and spermatogonia/oogonia, identified the regions that are unique in the tuna Vasa gene to avoid false detection of croaker Vasa gene highly homologous to the tuna Vasa gene, thus found that it can be used as an identification marker for a spermatogonia/oogonia derived from tuna primordial germ cells. Moreover, in order to analyze tuna germ cells transplanted into the genital gland of the host, it is necessary to establish a method to distinguish between the Vasa genes of tuna and the host and to detect the tuna gene exclusively. However, the Vasa genes of fish are highly homologous in nucleotide sequence, and it was very difficult to design a PCR primer set that specifically detects tuna Vasa gene expression. Therefore, the inventors of the present application performed nested PCR, which can amplify DNA with high specificity starting with even a very small amount of DNA, and specifically detected the tuna Vasa gene. Additionally, the present inventors compared the tuna Vasa gene sequence with other *Percomorphi* Vasa gene sequences, and identified a restriction enzyme sequence that is contained in the tuna Vasa gene only. The combination of these nested PCR and the restriction enzyme treatment established a method for detecting the tuna Vasa gene with increased reliability. This time, the present inventors produced many candidate peptide fragments that are specific for the tuna Vasa protein using amino acid sequence information of the tuna Vasa protein, and generated many monoclonal antibodies using such candidate peptide fragments as immunogens, found monoclonal antibodies that specifically bind to germ cells derived from a tuna exclusively, thus accomplishing the present invention.

Thus, the present invention relates to: (1) an anti-tuna Vasa antibody or fragment thereof, wherein the antibody is produced by using an antigen comprising one or more peptide fragments selected from the peptide fragment group consisting of amino acid sequences shown in any one of SEQ ID NOs: 1 to 9 in the sequence listing, the antibody specifically binds to a tuna vasa gene product, but does not bind to a vasa gene product from other fish species, (2) an anti-tuna Vasa antibody or fragment thereof, wherein the antibody is produced by using an antigen comprising one or more peptide fragments selected from the peptide fragment group consisting of amino acid sequences shown in any one of SEQ ID NOs: 1 to 9 in the sequence listing, the antibody specifically recognizes a germ cell derived from a tuna, but does not recognize a somatic cell derived from a tuna and a cell derived from other fish species, (3) the anti-tuna Vasa antibody or fragment thereof according to (1) or (2) mentioned above, wherein the antibody is produced by using an antigen comprising a peptide fragment consisting of the amino acid sequence shown in SEQ ID NO: 1 in the sequence listing, (4) the anti-tuna Vasa antibody or fragment thereof according to (2) or (3) mentioned above, wherein the germ cell derived from a tuna is a primordial germ cell, a spermatogonium, an oogonium or an oocyte, (5) the anti-tuna Vasa antibody or fragment thereof according to any one of (1) to (4) mentioned above, wherein the tuna is a bluefin tuna, and (6) the anti-tuna Vasa antibody or fragment thereof according to any one of (1) to (5) mentioned above, wherein the antibody is a monoclonal antibody.

Also, the present invention relates to: (7) a hybridoma having ability to produce the monoclonal antibody according to (6) mentioned above, (8) the hybridoma according to (7) mentioned above, wherein the hybridoma is vasa-C57Z 6H-7E (NITE BP-647) or vasa-C57Z 8A-11A (NITE BP-646), (9) a kit for detecting a germ cell derived from a tuna, wherein the kit comprises a labeled anti-tuna Vasa antibody or a labeled anti-tuna Vasa antibody fragment resulting from labeling the anti-tuna Vasa antibody or fragment thereof according to any one of (1) to (6) mentioned above, (10) a method for detecting a germ cell derived from a tuna, wherein the germ cell is transplanted into a heterologous recipient fish, the method comprises contacting the anti-tuna Vasa antibody or fragment thereof according to any one of (1) to (6) mentioned above, with a sample cell, and detecting a tuna Vasa protein expressed in the cell.

### Advantageous Effects of Invention

To examine whether donor fish-derived germ cells transplanted by surrogate fish technique proliferate and mature in the heterologous recipient fish gonad, it is necessary to use, as an indicator, a trait that is specifically expressed in germ cells and distinguishable between the recipient fish and the donor fish. A germ cell-specific gene, the Vasa gene is specific for primordial germ cells, spermatogonia, oogonia and/or oocytes, and its expression is not found in somatic cells. According to the present invention, a germ cell derived from a tuna can be reliably and simply distinguished among highly conserved Vasa gene sequences in fish, by using an anti-tuna Vasa monoclonal antibody capable of specifically detecting tuna germ cells, without performing sequence analysis, and, as result, culture and breeding of tuna can be efficiently carried out.

### Brief Description of Drawings

[Figure 1] Figure 1 shows the result of a comparison of Vasa amino acid sequences of bluefin tuna and other fish (black skipjack, skipjack, chub mackerel, blue mackerel, round frigate mackerel and frigate mackerel). In the figure, bold line indicates nine portions of amino acid sequence with low homologies in the comparison between bluefin tuna and other fish.
[Figure 2] Figure 2 shows the result of a Western blotting analysis that confirmed protein expression containing the four expressed peptide sequences in the supernatant of lysed bacterial sample.
[Figure 3] Figure 3 shows SDS-PAGE of the purified protein-eluted fractions of the expressed protein.
[Figure 4] Figure 4 shows the confirmation of expressed protein concentrations after concentration.
[Figure 5] Figure 5 shows the result of primary screening after cell fusion process. No. 6 (vasa-C57Z 6H-7E) and No. 8 (vasa-C57Z 8A-11A) showed strong responses to BFvasa14 (a) and mixed four BSA-crosslinked peptides (b), but showed no reaction to recombinant chub mackerel protein antigen (c).
[Figure 6] Figure 6 shows the result of screening on the hybridoma vasa-C57Z 6H-7E and the hybridoma vasa-C57Z 8A-11A by ELISA using each of the BSA-crosslinked peptides as an antigen one by one. They showed responses to the peptide of SEQ ID NO: 1.
[Figure 7] Figure 7 shows the result of immunohistological staining of germ cells derived from bluefin tuna and mackerel. The ovary (a) and testis (b) of bluefin tuna are stained brown with DAB, showing the binding to a monoclonal antibody of the present invention, while the ovary (c) and testis (d) of mackerel are not stained, showing no binding to the monoclonal antibody of the present invention.

### Best Mode of Carrying Out the Invention

An anti-tuna Vasa antibody of the present invention is not particularly limited, as long as it is an antibody produced by using an antigen, comprising one or more peptide fragments selected from peptide fragment group consisting of amino acid sequences shown in any one of SEQ ID NOs : 1 to 9 in the sequence listing (hereinafter, also referred to as tuna Vasa peptide fragment), wherein the antibody specifically binds to a tuna vasa gene product, but does not bind to a vasa gene product from another fish species, or wherein the antibody specifically recognizes a germ cell derived from a tuna, but does not recognize a somatic cell derived from a tuna and a cell derived from another fish species. "Tuna", as to the present invention, is a generic name for fish of *Percomorphi Scombroidei Scombridae Thunnus,* and includes specifically bluefin tuna, bigeye tuna, southern bluefin tuna, yellowfin tuna, albacore tuna, blackfin tuna, longtail tuna, and most preferably bluefin tuna. The above description "specifically recognizes a germ cell derived from a tuna, but does not recognize a somatic cell derived from a tuna and a cell derived from another fish species", as to the present invention, means to specifically bind to a Vasa gene product expressed in tuna germ cells, e.g. primordial germ cells, spermatogonia, oogonia and/or oocytes, but not to specifically bind to substance present in tuna somatic cells, or germ cells, e.g. primordial germ cell, spermatogonia, oogonia and/or oocytes of another fish that is not tuna.

Examples of antibodies of the present invention include, monoclonal antibodies, polyclonal antibodies, single chain antibodies, bifunctional antibodies, which can recognize two epitopes at the same time, etc., but monoclonal antibodies are particularly preferable due to their specificity for the recognition sites. The immunoglobulin classes of the antibodies are not particularly limited, and may be any of the isotypes IgG, IgM, IgA, IgD, IgE, etc., but IgG is preferable. Moreover, antibodies of the present invention may be a whole antibody or an antibody fragment, as long as it can specifically recognize a tuna Vasa gene product. Specific examples of the antibody fragment include, antibody fragments such as Fab fragments and F(ab')₂ fragments, CDR, multifunctional antibodies, single chain antibodies (ScFv), etc. For example, Fab fragments can be prepared by treating antibodies with papain, and F(ab')₂ fragments with pepsin. Moreover, antibodies of the present invention are not particularly limited by their origin, but may be, of mouse, rat, rabbit, or chicken origin, but monoclonal antibodies of mouse origin are preferable due to the easiness of their production.

Antigens used for the production of anti-tuna Vasa antibodies, particularly anti-tuna Vasa monoclonal antibodies of the present invention are not particularly limited as long as they comprise one or more peptide fragments selected from the peptide fragment group consisting of amino acid sequences shown in TSTITLTSRTSS (SEQ ID NO: 1), FWNTNGGEFG (SEQ ID NO: 2), CRMDQSEFNG (SEQ ID NO: 3), DNGMRENGYRG (SEQ ID NO: 4), GFSQGGDQGGRGGF (SEQ ID NO: 5), TRGEDKDPEKKDDSD (SEQ ID NO: 6), ADGQLARSLV (SEQ ID NO: 7), PATTGFNPPRKN (SEQ ID NO: 8) or RGSFQDNSVKSQPAVQTAADDD (SEQ ID NO: 9) in the amino acid sequences of a tuna Vasa protein (SEQ ID NO: 10). Among these, those comprising the peptide fragment consisting of the amino acid sequence of TSTITLTSRTSS (SEQ ID NO: 1) are preferable. Moreover, the peptide fragments mentioned above may be consisted of an amino acid sequence in which one or several amino acids have been deleted, substituted or added in an amino acid sequence shown in any one of SEQ ID NOs: 1 to 9. Furthermore, in order to increase antigenicity, a carrier protein such as KLH (Keyhole Limpet Hemocyanin), BSA (Bovine Serum Albumin), OVA (Ovalbumin) may be attached. For example, by introducing cysteine at the N- or C-terminus of a peptide fragment consisting of an amino acid sequence shown in any one of SEQ ID NOs: 1 to 9, maleimide-activating it via the SH group in the cysteine, and coupling a carrier protein to it, a cross-linked peptide can be produced and used for immunologic sensitization. Moreover, in order to efficiently raise antibodies against the peptide fragments mentioned above, an adjuvant such as Freund complete adjuvant (FCA) and Freund incomplete adjuvant (FIA) may be used upon immunologic sensitization, as needed. Examples of production methods of the peptide fragments mentioned above include chemical synthesis methods such as the Fmoc method (fluorenylmethyl oxycarbonyl method), tBoc method (t-butyloxy carbonyl method), amino acid sequence information-based synthesis methods using various peptide synthesizers commercially available, production methods by expressing a part or all of a tuna Vasa gene using phage or cells such as *E. coli,* actinomyces, lactobacilli, yeasts, and cultured cells.

Examples of preparation methods of an anti-tuna Vasa antibody of the present invention include methods of administering the peptide fragments mentioned above as antigens to an animal (preferably other than human), for example, using a conventional protocol, and specific examples include methods of administering the peptide fragments mentioned above as antigens to a mammal such as rat, mouse, rabbit, etc., to immunize it. Typically, antigens can be administered intravenously, subcutaneously or intraperitoneally. Immunization intervals are not particularly limited, but preferably from several days to several weeks, more preferably 1 to 4 weeks. The number of immunization is preferably 1 to 10 times, and more preferably 1 to 5 times. Antibody producing cells are collected 1 to 60 days, preferably 1 to 14 days after the day of last immunization. Antibody producing cells are preferably cells derived from splenic cells, lymph node cells, or peripheral blood cells, and more preferably splenic cells or local lymph node cells.

To prepare an anti-tuna Vasa monoclonal antibody of the present invention, any method can be used, including hybridoma method (Nature 256, 495-497, 1975), trioma method, human B-cell hybridoma method (Immunology Today 4, 72, 1983) and EBV-hybridoma method (MONOCLONAL ANTIBODIES AND CANCER THERAPY, pp. 77-96, Alan R. Liss, Inc., 1985), in which antibody is produced in culture of continuous cell line. Production of monoclonal antibody-producing hybridoma using the hybridoma method mentioned above can be done by fusing the antibody producing cells mentioned above and myeloma cells. As the myeloma cells mentioned above, generally available cell lines such as mouse- or rat-derived cells can be used. The antibody producing cells and myeloma cells mentioned above are preferably of the same animal species, and preferably have the properties of being not able to survive in the HAT selection medium (containing hypoxanthine, aminopterin, and thymidine) when not fused, and of being able to survive as positive hybridoma only when fused with antibody producing cells. Specific examples include mouse myeloma cell lines such as P3-X63-Ag8-U, NSI/1-Ag4-1, NSO/1, and rat myeloma cell lines such as YB2/0.

Hybridoma of the present invention are not particularly limited, as long as they have ability to produce an anti-tuna Vasa monoclonal antibody of the present invention, but specific examples include vasa-C57Z 6H-7E and vasa-C57Z 8A-11A described in the examples below. The hybridoma vasa-C57Z 6H-7E and vasa-C57Z 8A-11A mentioned above were respectively deposited to Patent Microorganisms Depositary, National Institute of Technology and Evaluation, 2-5-8 Kazusakamatari, Kisarazushi, Chiba, Japan, under the accession number of NITE BP-647 and NITE BP-646 on September 24, 2008.

A Kit to detect an germ cell derived from a tuna of the present invention is not particularly limited, as long as it comprises an labeled antibody or labeled antibody fragment, in which an anti-tuna Vasa antibody of the invention or fragment thereof is labeled, or it comprises a labeled antibody (labeled secondary antibody) or labeled substance that recognizes the anti-tuna Vasa antibody of the present invention. A labeling substance used to generate the labeled antibody or labeled antibody fragment mentioned above is not particularly limited, as long as it is capable of producing a signal that enables the detection of a germ cell derived from a tuna by itself or by reacting with another substance. Examples include enzymes such as peroxidase, alkaline phosphatase, β-D-galactosidase, glucose oxidase, catalase or urease; fluorescent substances such as fluorescein isothiocyanate (FITC), phycoerythrin or tetramethyl rhodamine isothiocyanate; luminescent substances such as luminol, dioxetane, acridinium salts; radioactive substances such as ³H, ¹⁴C, ¹²⁵I or ¹³¹I. When the labeling substance is an enzyme, it is preferable to include a substrate as needed, and coloring agent, fluorescent agent, luminescent agent, etc., if necessary, to measure the activity. By using a detection kit comprising a labeled antibody of the present invention, the presence of a germ cell derived from a tuna can be detected, and the localization and density in vivo can be investigated.

A method for detecting a germ cell derived from a tuna transplanted into a heterologous recipient fish of the present invention is not particularly limited, as long as it is a method for detecting a germ cell derived from a tuna transplanted into a heterologous recipient fish, wherein the method comprises contacting the anti-tuna Vasa antibody or fragment thereof of the present invention with a sample, and detecting the antibody bound to a tuna Vasa protein expressed in cells in the sample. The method is also useful as a method to evaluate proliferation and/or maturation of the germ cell derived from a tuna in the heterologous recipient fish. In a detection method of the present invention, a method for detecting the antibody bound to a tuna Vasa protein may be, for example, an immunological assay, such as ELISA, RIA, Western blotting, immunoprecipitation, immunohistological staining, plaque technique, spotting, hemagglutination test, or Ouchterlony method. By using a detection method of the present invention, for example, transplantation of primordial germ cells isolated from a tuna into early embryo of a heterologous recipient fish, such as croaker, mackerel, black skipjack, schnapper, whose seed production is more simple and efficient than tuna, preferably transplantation into the abdominal cavity of a heterologous recipient fish at an early developmental stage can be performed and thereby differentiation of the aforementioned primordial germ cells into the germ cell lineage can be induced. As result, the primordial germ cells derived from a tuna are induced to differentiate into spermatogonia, oogonia or oocytes in the heterologous recipient fish individuals, and further to differentiate into ova or sperms, which enables reproduction and breeding of tuna.

The present invention is described more specifically with examples below. These examples are not intended to limit the technical scope of the present invention.

### Example 1

### (Production of monoclonal antibodies)

### [Antigen preparation 1; preparation of KLH-crosslinked peptides]

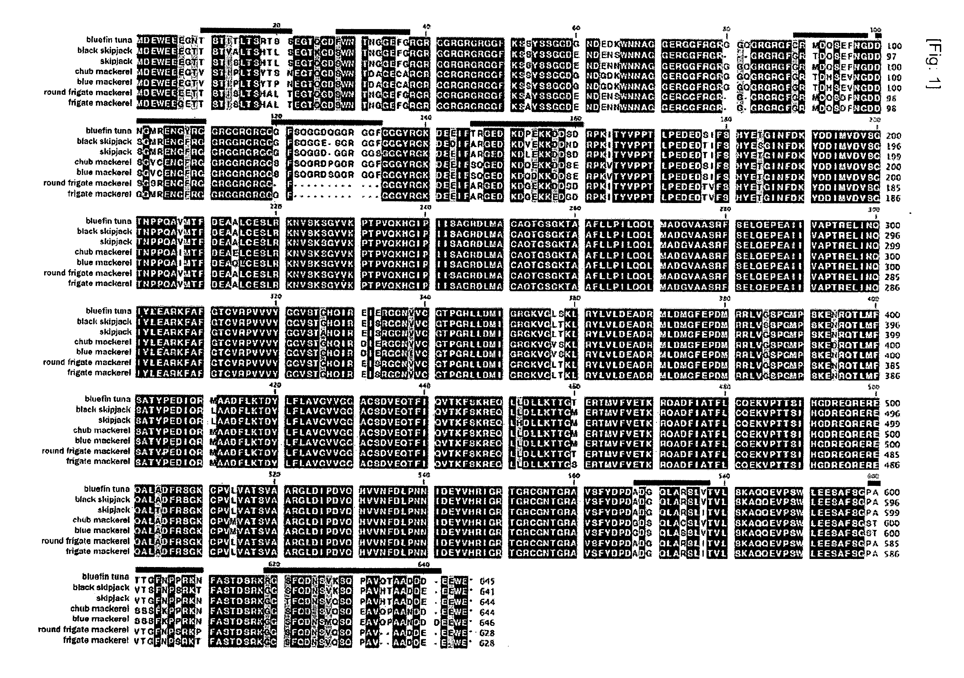

A comparison of Vasa amino acid sequences of bluefin tuna (SEQ ID NO: 10) and those of other fish (black skipjack, skipjack, chub mackerel, blue mackerel, round frigate mackerel and frigate mackerel) was made (Figure 1). As a result, 9 regions of amino acid sequence, TSTITLTSRTSS (SEQ ID NO: 1), FWNTNGGEFG (SEQ ID NO: 2), CRMDQSEFNG (SEQ ID NO: 3), DNGMRENGYRG (SEQ ID NO: 4), GFSQGGDQGGRGGF (SEQ ID NO: 5), TRGEDKDPEKKDDSD (SEQ ID NO: 6), ADGQLARSLV (SEQ ID NO: 7), PATTGFNPPRKN (SEQ ID NO: 8) and RGSFQDNSVKSQPAVQTAADDD (SEQ ID NO: 9) were identified as the regions that are particularly different in sequence between bluefin tuna and other fish. Then, the peptide of SEQ ID NO: 3 and the peptides of SEQ ID NO: 1, 2 and 4 with an added cysteine necessary for KLH crosslinking at C-terminus were synthesized by Fmoc solid-phase synthesis method. The resulting products were purified by HPLC. To the cysteine residue of each of the purified peptides, maleimide-activated KLH (Pierce) was conjugated as a carrier peptide following the protocol. These were used as antigens for immunization in the production of monoclonal antibodies as described below.

### [Antigen preparation 2; preparation of BFvasa14 recombinant protein] 1. Construction of expression vector

In order to increase the immunogenicity, sequence near the peptide region including the 4 amino acid sequences mentioned above was selected for the preparation of an antigen protein. RT-PCR was performed using the forward primer (5'-GCCGGGATCCAGCACTATTACACTAACCAGCCGC-3' : SEQ ID NO: 12) and the reverse primer (5'-GCCGAAGCTTGCTGAAACCTCCTCCTCTTCCTCT-3': SEQ ID NO: 13) with added restriction enzyme sites of BamHI and HindIII, and the tuna vasa gene (SEQ ID NO: 11) as a template. A cDNA fragment of 333 bp was amplified. The polymerase TaKaRa LATaq was used. The cDNA fragment was inserted into BamHI-HindIII sites in pColdIDNA (Takara bio Co., Ltd.), a cold shock expression vector, ligated with T4DNA ligase (Promega), and an E. coli expression vector was constructed. An E. coli host for expression, BL21(DE3) competent cells (Invitrogen life technologies) were transformed with the constructed expression vector. With E. coli from an obtained single colony, the liquid LB medium containing 100 µg/mL of ampicillin was inoculated. The culture was shaken for 16 hours at 37°C. Subsequently, liquid LB medium was inoculated with 1/100 total volume of the saturated bacterial culture, and the culture was shaken for 2 hours at 37°C. This culture was left at 15°C for 30 minutes, and then IPTG was added to the final concentration of 1 mM. The culture was shaken for 24 hours at 15°C.

### 2. Construction of expression vector and protein expression

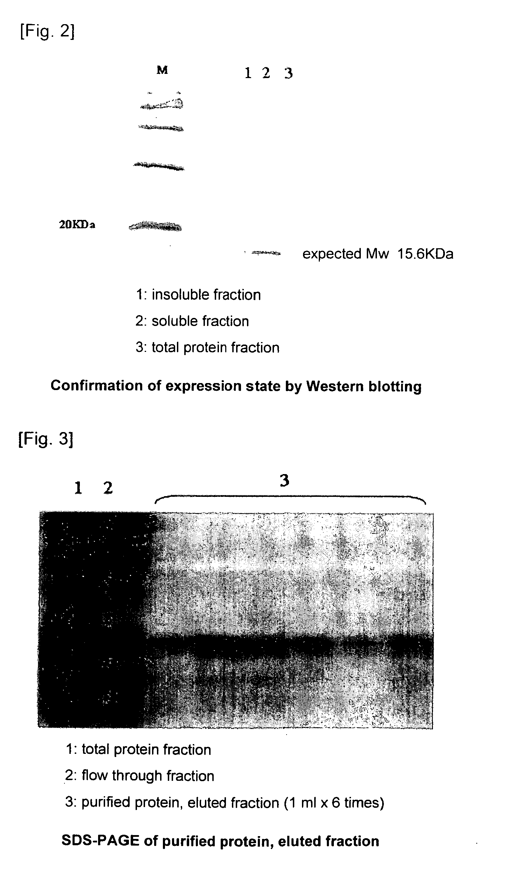

After shaking for 24 hours, 1.5 mL of the bacterial culture mentioned above was centrifuged at 13,000 rpm, 4°C for 5 minutes to harvest bacterial cells. The cells were lysed with B-PER (Bacterial Protein Extraction Reagent; PIERCE Thermo Scientific) and centrifuged at 13,000 rpm, 4°C for 15 minutes. The supernatant and precipitate were both recovered. The recovered supernatant and precipitate were dissolved into 10 µL of sample buffer (0.5M Tris-HC1 (pH 6.8) 1 mL, 10% SDS 2 mL, β-mercaptoethanol 0.6 mL, glycerol 1 mL, D.W. 5.4 mL, 1% BPB 100 µL/10 mL). SDS-PAGE was performed with 15% polyacrylamide gel at 250V, 20 mA. After the end of electrophoresis, Western blotting was performed using the nitrocellulose membrane Hybond-ECL (GE health care bioscience Co., Ltd.) at 80V, 250 mA. Because a histidine tag (6 His tag) is added to the expressed protein, the expression states were confirmed by color development with the substrate NBT/BCIP using Ni-NTA AP conjugate (QIAGEN) as an antibody against the protein of interest. As a result, it was found that the supernatant of the lysed samples contained the protein of interest, expressing in a soluble state (see Figure 2).

### 3. Protein purification and concentration

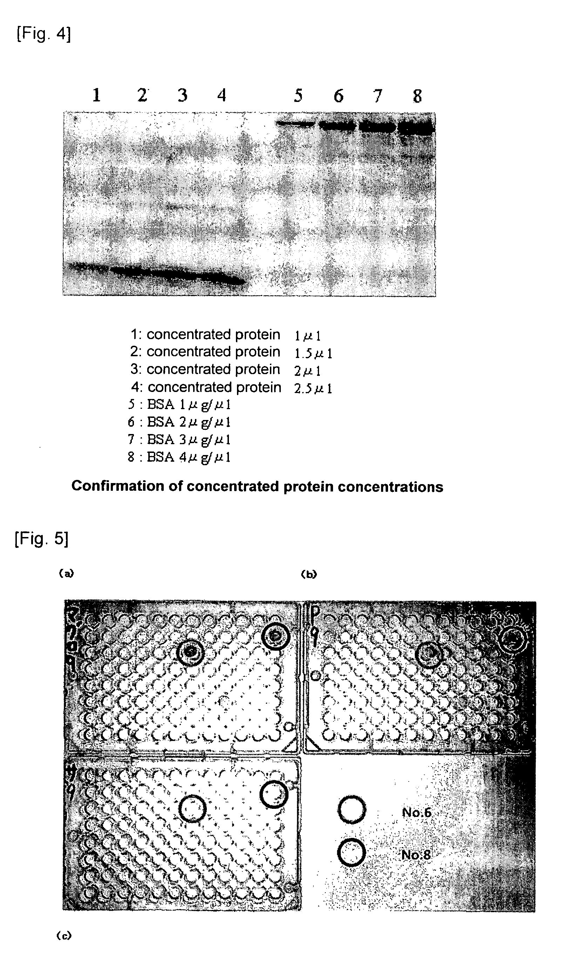

To obtain expressed protein to purify, 6L of liquid LB medium containing 100 µg/mL of ampicillin was inoculated with 60 mL of saturated bacterial culture. The culture was shaken in an incubator at 37°C for 2 hours. This culture was left at 15°C for 30 minutes, and then IPTG was added to the final concentration of 1 mM. The culture was shaken at 15°C for 24 hours. Bacterial cells were harvested by centrifuge at 4,000 rpm, 4°C for 20 minutes, lysed using TALON xTractor buffer kit (Clontech) and centrifuged at 13,000 rpm, 4°C for 20 minutes to get a soluble fraction. The solution of this fraction was mixed with TALON metal affinity resin (Metal Affinity Resins; Clontech), resin beads coupled with cobalt ions, to adsorb the protein of interest. These beads were loaded into a column. Protein adhered nonspecifically to the beads were removed by applying 2 mL of buffer (50 mM NaH₂PO₄, 300 mM NaCI, 60 mM imidazole, pH 7.0) 6 times, and the protein of interest was eluted by applying 1 mL of elution buffer (50 mM NaH₂PO₄, 300 mM NaCl, 150 mM imidazole pH 7.0) 6 times (see Figure 3). The protein solution after the purification was subjected to the ultrafiltration using Amicon Ultra-15 Ultracel-3K (MILLIPORE) to concentrate into 1 mg/1.5 mL. This purified protein was designated as BFvasa14 recombinant protein, and 1 mg was used for monoclonal antibody production (see Figure 4).

### [Mouse immunization]

Mixed four KLH-crosslinked peptide solution was prepared as 1 mL antigen solution by mixing 250 µL each of four KLH-crosslinked peptides prepared as mentioned above. BFvasa14 recombinant protein solution 300 µL was also used as an antigen solution. The mixed four KLH-crosslinked peptide solution mentioned above was emulsified by adding 1 mL of Freund complete adjuvant (Pierce). The emulsified mixed four KLH-crosslinked peptide solution was divided into equal aliquots and subcutaneously administered to 4 animals of C57BL6 mice (purchased from Japan SLC) of 9 weeks old on their back for priming. Separately, 300 µL of BFvasa14 recombinant protein solution was emulsified by adding 300 µL of Freund complete adjuvant (Pierce). The emulsified BFvasa14 recombinant protein solution was divided into equal aliquots and subcutaneously administered to 2 animals of C57BL6 mice of 9 weeks old on their back.

### [Cell fusion]

Lymph node cells were aseptically removed from the 6 mice mentioned above 14 days after the priming mentioned above. The lymph node cells were mixed and fused with P3-X63-Ag8-U mouse myeloma cells stocked at Tokyo University of Marine Science and Technology in the presence of 50% polyethylene glycol. The mixed cells were diluted in microtiter plates containing the HAT medium. After 8 days of culturing, the presence or absence of antibody in antisera and conditioned media was detected by enzyme-linked immunoassay (ELISA). As immunogens for screening, the BFvasa14 recombinant protein mentioned above and mixed four BSA-crosslinked peptides were used. The peptides were crosslinked with BSA instead of KLH to remove antibodies that bind to KLH alone. The plates were prepared by diluting the antigens for screening mentioned above with PBS to 5 µg/mL each. As blank, plates were prepared in a similar way as above by diluting a recombinant protein antigen from chub mackerel with PBS to 10 µg/mL (see Figure 5(c)). By assaying for each antigen (959 wells in total), total 14 strains were found as strains that respond to BFvasa14 recombinant protein alone; strains that respond to BFvasa14 recombinant protein and BSA-crosslinked peptides; and strains that is responsive to BFvasa14 recombinant protein, BSA-crosslinked peptides and the recombinant protein antigen from chub mackerel. Two strains, No. 6 and No. 8, which show no response to chub mackerel, but strong response to BFvasa14 recombinant protein and BSA-crosslinked peptides, were selected (see Figure 5(a) and (b)). No. 6 was designated as vasa-C57Z 6H-7E, and No. 8 as vasa-C57Z 8A-11A. Tables 1 to 3 below show the results of primary screening after the cell fusion treatment which lead to the selection of the hybridoma.

As shown in Figure 5, No. 6 (vasa-C57Z 6H-7E) showed strong responses to BFvasa14 (0.638) and to mixed four BSA-crosslinked peptides (0.247); and No. 8 (vasa-C57Z 8A-11A) to BFvasa14 (0.624) and to mixed four BSA-crosslinked peptides (0.410), while they showed no response to the recombinant protein antigen from chub mackerel (0.062 and 0.063 respectively).

**[Table 1]**

| BFvasa14 recombinant peptide plate (5µg/mL) | | | | | | | | | | | |
|---|---|---|---|---|---|---|---|---|---|---|---|
| 0.066 | 0.061 | 0.052 | 0.066 | 0.060 | 0.057 | 0.062 | 0.063 | 0.063 | 0.063 | 0.064 | 0.067 |
| 0.084 | 0.064 | 0.054 | 0.061 | 0.054 | 0.064 | 0.064 | 0.064 | 0.065 | 0.064 | 0.064 | 0.624 |
| 0.063 | 0.052 | 0.050 | 0.076 | 0.063 | 0.062 | 0.638 | 0.109 | 0.066 | 0.064 | 0.063 | 0.062 |
| 0.063 | 0.052 | 0.054 | 0.052 | 0.064 | 0.064 | 0.063 | 0.062 | 0.052 | 0.096 | 0.063 | 0.080 |
| 0.063 | 0.061 | 0.060 | 0.053 | 0.067 | 0.053 | 0.052 | 0.059 | 0.052 | 0.062 | 0.063 | 0.062 |
| 0.065 | 0.063 | 0.065 | 0.104 | 0.065 | 0.061 | 0.062 | 0.060 | 0.198 | 0.054 | 0.080 | 0.052 |
| 0.062 | 0.063 | 0.063 | 0.064 | 0.063 | 0.060 | 0.071 | 0.062 | 0.074 | 0.062 | 0.062 | 0.068 |
| 0.066 | 0.066 | 0.067 | 0.066 | 0.065 | 0.066 | 0.067 | 0.064 | 0.065 | 0.065 | 0.069 | 0.064 |

**[Table 2]**

| Mixed four BSA-crosslinked peptide plate (5µg/mL) | | | | | | | | | | | |
|---|---|---|---|---|---|---|---|---|---|---|---|
| 0.064 | 0.062 | 0.063 | 0.063 | 0.064 | 0.070 | 0.062 | 0.063 | 0.063 | 0.062 | 0.065 | 0.061 |
| 0.154 | 0.058 | 0.070 | 0.064 | 0.063 | 0.067 | 0.066 | 0.063 | 0.062 | 0.063 | 0.064 | 0.410 |
| 0.062 | 0.065 | 0.062 | 0.062 | 0.065 | 0.064 | 0.247 | 0.122 | 0.067 | 0.063 | 0.062 | 0.061 |
| 0.062 | 0.062 | 0.064 | 0.065 | 0.066 | 0.063 | 0.063 | 0.062 | 0.063 | 0.065 | 0.061 | 0.062 |
| 0.066 | 0.062 | 0.061 | 0.063 | 0.064 | 0.061 | 0.061 | 0.067 | 0.061 | 0.060 | 0.066 | 0.064 |
| 0.063 | 0.062 | 0.062 | 0.063 | 0.070 | 0.061 | 0.072 | 0.063 | 0.226 | 0.068 | 0.062 | 0.066 |
| 0.061 | 0.062 | 0.064 | 0.063 | 0.061 | 0.061 | 0.097 | 0.062 | 0.062 | 0.061 | 0.062 | 0.071 |
| 0.070 | 0.067 | 0.065 | 0.064 | 0.063 | 0.065 | 0.064 | 0.065 | 0.080 | 0.070 | 0.064 | 0.064 |

**[Table 3]**

| Chub mackerel plate(10µg/mL) | | | | | | | | | | | |
|---|---|---|---|---|---|---|---|---|---|---|---|
| 0.064 | 0.064 | 0.065 | 0.065 | 0.064 | 0.064 | 0.063 | 0.072 | 0.063 | 0.064 | 0.063 | 0.077 |
| 0.073 | 0.076 | 0.065 | 0.063 | 0.063 | 0.065 | 0.064 | 0.064 | 0.066 | 0.064 | 0.063 | 0.063 |
| 0.063 | 0.063 | 0.063 | 0.064 | 0.064 | 0.064 | 0.062 | 0.063 | 0.063 | 0.062 | 0.062 | 0.063 |
| 0.066 | 0.065 | 0.065 | 0.065 | 0.065 | 0.065 | 0.065 | 0.063 | 0.065 | 0.063 | 0.063 | 0.064 |
| 0.063 | 0.063 | 0.064 | 0.063 | 0.063 | 0.068 | 0.063 | 0.062 | 0.063 | 0.062 | 0.062 | 0.062 |
| 0.065 | 0.064 | 0.068 | 0.064 | 0.063 | 0.062 | 0.064 | 0.063 | 0.066 | 0.063 | 0.062 | 0.063 |
| 0.064 | 0.064 | 0.065 | 0.066 | 0.063 | 0.064 | 0.079 | 0.063 | 0.064 | 0.064 | 0.064 | 0.066 |
| 0.068 | 0.067 | 0.068 | 0.066 | 0.073 | 0.068 | 0.067 | 0.066 | 0.068 | 0.065 | 0.066 | 0.069 |

### [Reexamination of selection of fusion cells]

Cells were captured into 48 well microplates (Falcon) from the positive wells of original strains and cultured. Antibody titers in the supernatants were measured again. The strains showing response to the antigens were subjected to the limiting dilution cloning in cloning medium.

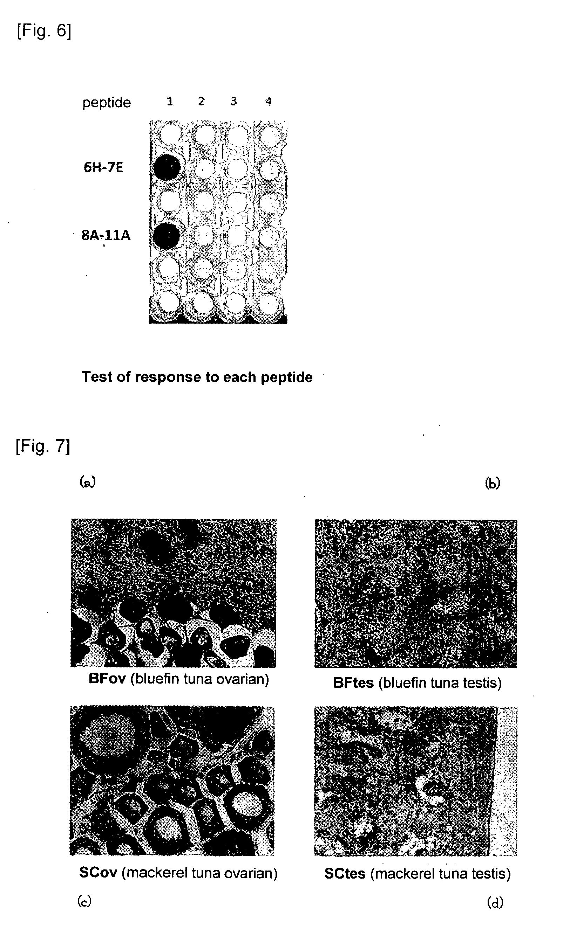

Screening by ELISA was carried out using, as an antigen, each one of the BSA-crosslinked peptides mentioned above for the hybridoma vasa-C57Z 6H-7E and the hybridoma vasa-C57Z 8A-11A, respectively. Both showed strong responses when using, as an antigen, the peptide in which the peptide fragment consisting of amino acid sequence shows in SEQ ID NO: 1 was BSA-crosslinked. The results are shown in Figure 6.

The monoclonal antibodies of the present invention were taken from conditioned media from the cultures in which the hybridoma vasa-C57Z 6H-7E and vasa-C57Z 8A-11A of the present invention mentioned above had been cultured in RPMI-1640 medium containing 10% fetal bovine serum etc., at 37°C for 4 to 5 days. The subclass of the antibodies was confirmed using Mouse Monoclonal Antibody Isotyping Test Kit (product cord: MMT1, Serotec); vasa-C57Z 6H-7E produces IgG2bκ chain, and vasa-C57Z 8A-11A produces IgG3κ chain.

### Example 2

### [Immunohistological staining of germ cells derived from bluefin tuna and mackerel]

Deparaffinization of each sample of bluefin tuna paraffin-embedded tissue sections (bluefin tuna ovarian tissue and bluefin tuna testis tissue (obtained from Nakatani Suisan), and mackerel ovarian tissue and mackerel testis tissue (derived from offshore of Tateyama, Chiba)) fixed with 4% PFA/PBS fixation liquid was performed with xylene-ethanol solutions and hydration treatment. The resulting section samples were treated with antigen retrieval agent by immersing in the antigen retrieval solution HistoVT One (Nakarai tesque) at 90°C for 20 minutes, washing with PBS/0.1% Tween 20, blocking at room temperature with 2% BSA/PBS for 30 minutes. As a primary antibody, 0.1 mL of diluted supernatant from the hybridoma vasa-C57Z 6H-7E (antibody dilution: 1 and 10 times) was added dropwise onto section samples. The samples were incubated at 4°C overnight, washed 3 times with PBS/0.1% Tween 20 for 5 minutes, then blocked at room temperature for 30 minutes using normal horse serum for blocking (ImmPRESS kit, Vector). As a secondary antibody, ImmPRESS REAGENT anti-mouse IgG antibody was added dropwise onto section samples. The samples were incubated at room temperature for 1 hour, washed 3 times with PBS/0.1% Tween 20 for 5 minutes, then the section samples were stained with DAB reagent for 5 to 10 minutes.

The stained section samples mentioned above were observed under BX50 (Olympus). Figure 7 shows examples of immunostaining of germ cells in bluefin tuna ovarian tissue and testis tissue, and mackerel ovarian tissue and testis tissue with the antibody (antibody dilution: 10 times) produced by the hybridoma vasa-C57Z 6H-7E mentioned above. It was shown that the antigen protein expressed in the bluefin tuna ovarian tissue and testis tissue was stained brown (see Figure 7 (a) and Figure 7 (b)), while germ cells of mackerel were not stained (see Figure 7 (c) and Figure 7 (d)). Thus, it was shown that, by an immunostaining method using the monoclonal antibody of the present invention, an antigen protein expressed in bluefin tuna germ cells that specifically binds to a monoclonal antibody of the present invention can be detected.

### Accession numbers

NITE BP-646
NITE BP-647

## Claims

1. An anti-tuna Vasa antibody or fragment thereof, wherein the antibody is produced by using an antigen comprising one or more peptide fragments selected from the peptide fragment group consisting of amino acid sequences shown in any one of SEQ ID NOs: 1 to 9 in the sequence listing, the antibody specifically binds to a tuna vasa gene product, but does not bind to a vasa gene product from other fish species.

2. An anti-tuna Vasa antibody or fragment thereof, wherein the antibody is produced by using an antigen comprising one or more peptide fragments selected from the peptide fragment group consisting of amino acid sequences shown in any one of SEQ ID NOs: 1 to 9 in the sequence listing, the antibody specifically recognizes a germ cell derived from a tuna, but does not recognize a somatic cell derived from a tuna and a cell derived from other fish species.

3. The anti-tuna Vasa antibody or fragment thereof according to claim 1 or 2, wherein the antibody is produced by using an antigen comprising a peptide fragment consisting of the amino acid sequence shown in SEQ ID NO: 1 in the sequence listing.

4. The anti-tuna Vasa antibody or fragment thereof according to claim 2 or 3, wherein the germ cell derived from a tuna is a primordial germ cell, a spermatogonium, an oogonium or an oocyte.

5. The anti-tuna Vasa antibody or fragment thereof according to any one of claims 1 to 4, wherein the tuna is bluefin tuna.

6. The anti-tuna Vasa antibody or fragment thereof according to any one of claims 1 to 5, wherein the antibody is a monoclonal antibody.

7. A hybridoma having ability to produce the monoclonal antibody according to claim 6.

8. The hybridoma according to claim 7, wherein the hybridoma is vasa-C57Z 6H-7E (NITE BP-647) or vasa-C57Z 8A-11A (NITE BP-646).

9. A kit for detecting a germ cell derived from a tuna, wherein the kit comprises a labeled anti-tuna Vasa antibody or a labeled anti-tuna Vasa antibody fragment resulting from labeling the anti-tuna Vasa antibody or fragment thereof according to any one of claims 1 to 6.

10. A method for detecting a germ cell derived from a tuna, wherein the germ cell is transplanted into a heterologous recipient fish, the method comprises contacting the anti-tuna Vasa antibody or fragment thereof according to any one of claims 1 to 6 with a sample cell, and detecting a tuna Vasa protein expressed in the cell.
